# EUROPEAN PATENT SPECIFICATION

(11) **EP 3 928 718 B1**
(45) Date of publication and mention of the grant of the patent: **21.10.2026**
(21) Application number: 21181592.3
(22) Date of filing: 24.06.2021
(51) Int. Cl.: A61B 17/072

(54) **LINEAR STAPLING DEVICE WITH CONTINUOUSLY PARALLEL JAWS**
LINEARE KLAMMERVORRICHTUNG MIT KONTINUIERLICH PARALLELEN BACKEN
DISPOSITIF D'AGRAFAGE LINÉAIRE DOTÉ DE MÂCHOIRES PARALLÈLES EN CONTINU

(30) Priority: 25.06.2020 US 202016911825
(43) Date of publication of application: 29.12.2021
(73) Proprietor: Covidien LP, Mansfield, MA 02048 (US)
(72) Inventor: VADALI, K V S Manoj Kumar, 502032 Hyderabad (IN); FERNANDES, Roanit A., 500026 Hyderabad (IN)
(74) Representative: Zimmermann & Partner Patentanwälte mbB

(56) References cited:
- WO-A1-2016/044216
- WO-A2-2012/019063
- US-A1- 2018 168 574

## Description

### FIELD

This technology is generally related to surgical stapling devices and, more particularly, to surgical stapling devices that include a tool assembly including jaws in continuously parallel alignment.

### BACKGROUND

Surgical stapling devices for simultaneously cutting and stapling tissue are known in the art and are commonly used during surgical procedures to reduce the time required to perform the surgical procedure and to facilitate endoscopic access to a surgical site. Performing a surgical procedure endoscopically reduces the amount of trauma inflicted on a patient during a surgical procedure to minimize patient discomfort and reduce patient recovery times.

Typically, endoscopic stapling devices include a tool assembly having a first jaw, and a second jaw that can pivot in relation to the first jaw between an open or spaced position and a closed or clamped position. One of the first and second jaws supports a cartridge assembly that includes a plurality of staples and the other of the first and second jaws supports an anvil assembly that includes an anvil having staple deforming pockets that receive and deform legs of the staples when the staples are ejected from the cartridge assembly.

In known devices, each of the first and second jaws is in pivotal relation with one another about a pivot point or hinge. Pivoting the first and second jaws creates mutually inclined surfaces in the first and second jaws, thus creating inconsistent pressure to tissue therebetween. As a result, tissue may pushed toward a distal end of the first and second jaws upon closure of the jaws. US Patent Application US2018/168574 describes a surgical stapler whose configuration is convertible; PCT Application WO 2016/044216 describes a surgical stapler including a spring-loaded lift mechanism that adjusts automatically, and PCT Application WO 2012/019063 describes a surgical stapler which maintains the anvil and cartridge assemblies substantially parallel and opposite to one another.

### SUMMARY

In one aspect of the disclosure is provided a tool assembly for a surgical stapling device, comprising:
a first jaw defining a first surface;
a second jaw defining a second surface, the second jaw movable in relation to the first jaw between an open position and a clamped position in which the second surface is in juxtaposed alignment with the first surface;
an I-beam movable from a retracted position towards an advanced position to move the second jaw in relation to the first jaw from the open position to the clamped position;
at least two first pivoting links operably coupled to a first side of the first and second jaws; characterized in including:
   at least two second pivoting links operably coupled to a second side of the first and second jaws, the first pivoting links and the second pivoting links each configured to pivot in response to longitudinal advancement of the I-beam to move the second jaw between the open position and the clamped position,
   wherein the second surface of the second jaw is maintained in substantially parallel alignment with the first surface of the first jaw as the second jaw is moved between the open position and the clamped position.

Each of the first and second pivoting links may define a first pivoting projection engaged with the second jaw, a second pivoting projection engaged with the first jaw, and a connecting strut connecting the first pivoting projection with the second pivoting projection; preferably wherein the first pivoting projection projects in an opposite direction from the second pivoting projection.

The tool assembly may further include a proximally facing ramp surface formed at a proximal end portion of the second jaw, the I-beam engaging the proximal-facing surface of the ramp surface when the I-beam moves from its retracted position towards its advanced position to move the second jaw between the open position and the clamped position, wherein the ramp surface defines a slot and the I-beam includes a vertical strut, the slot configured to receive the vertical strut of the I-beam.

The second jaw may define a channel, the channel configured to receive a lower beam of the I-beam as the I-beam moves the second jaw between the open position and the clamped position.

The tool assembly may further include a mounting assembly secured to the first jaw, the mounting assembly including a leaf spring, the leaf spring configured to bias the second jaw towards the open position; preferably wherein the leaf spring is laterally offset from the I-beam.

The I-beam may include a distal-facing surface that defines a knife.

In a further aspect is provided a surgical stapling device, comprising: an elongate body having a proximal portion and a distal portion; and a reload assembly including a proximal body portion and a tool assembly as above, the proximal body portion adapted to be releasably coupled to the distal portion of the elongate body, and the tool assembly being supported on the proximal body portion, wherein: said first jaw supports an anvil, the anvil defining said first surface; said second jaw supports a cartridge assembly, the cartridge assembly including a staple cartridge defining said second surface and a plurality of staple pockets, the second jaw movable in relation to the first jaw between an open position and a clamped position in which the second surface of the staple cartridge is in juxtaposed alignment with the first surface of the anvil; and including: a drive member having a proximal portion and a distal portion, the distal portion of the drive member supporting said I-beam, the I-beam having a first beam, a second beam, and a vertical strut connecting the first beam with the second beam, the drive member movable from a retracted position towards an advanced position to move the second jaw in relation to the first jaw from the open position to the clamped position.

Each of the first and pivoting links may define a first pivoting projection engaged with the second jaw, a second pivoting projection engaged with the first jaw, and a connecting strut connecting the first pivoting projection with the second pivoting projection; preferably wherein the first pivoting projection projects in an opposite direction from the second pivoting projection.

The surgical stapling device may further include a proximally facing ramp surface formed at a proximal end portion of the second jaw, the I-beam engaging the proximal-facing surface of the ramp surface when the I-beam moves from its retracted position towards its advanced position to move the second jaw between the open position and the clamped position, wherein the ramp surface defines a slot and the I-beam includes a vertical strut, the slot configured to receive the vertical strut of the I-beam; preferably wherein the second jaw defines a channel, the channel configured to receive the lower beam of the I-beam as the I-beam moves the second jaw between the open position and the clamped position.

The surgical stapling device may further include a mounting assembly secured to the first jaw, the mounting assembly including a leaf spring, the leaf spring configured to bias the second jaw towards the open position; preferably wherein the leaf spring is laterally offset from the I-beam.

The I-beam may include a distal-facing surface that defines a knife.

The surgical stapling device may further including a plurality of staple forming pockets formed in the anvil, each of the staple forming pockets configured to be in juxtaposed alignment with a corresponding staple pocket of the cartridge assembly when the second jaw is in the clamped position.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various aspects and features of the disclosure are described with reference to the drawings wherein like numerals designate identical or corresponding elements in each of the several views and:
FIG. 1 is a side perspective view of exemplary aspects of the disclosed stapling device including a tool assembly in an open position;
FIG. 2 is an enlarged side perspective view of the indicated area of detail shown in FIG. 1;
FIG. 3 is an exploded side perspective view of the tool assembly of the stapling device shown in FIG. 1;
FIG. 4 is an enlarged side perspective view of the indicated area of detail shown in FIG. 3;
FIG. 5 is an enlarged perspective view of a channel of the tool assembly of the stapling device shown in FIG. 1;
FIG. 6 is a perspective view of a reload assembly supporting the tool assembly of FIG. 1;
FIG. 7 is an enlarged perspective view of the indicated area of detail shown in FIG. 6;
FIG. 8 is a cross-sectional view taken along section line 8-8 of FIG. 7;
FIG. 9 is a rear perspective view of the tool assembly of the stapling device of FIG. 1 in an open configuration;
FIG. 10 is a cross-sectional view take along section line 10-10 of FIG. 9;
FIG. 11 is a cross-sectional view take along section line 11-11 of FIG. 2;
FIG. 12 is an enlarged side cross-sectional view of the indicated area of detail shown in FIG. 11;
FIG. 13 is a front perspective view of the tool assembly of the stapling device of FIG. 1 in a clamped configuration;
FIG. 14 is a bottom perspective view of the tool assembly of the stapling device of FIG. 13 in the clamped configuration;
FIG. 15 is a cross-sectional view take along section line 15-15 of FIG. 13; and
FIG. 16 is an enlarged side view of the indicated area of detail shown in FIG. 15.

### DETAILED DESCRIPTION

The disclosed surgical stapling device will now be described in more detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as horizontal, vertical, distal, proximal, and similar terms are used to assist in understanding the description and are not intended to limit the disclosure.

As used herein, the term "distal" refers to the portion that is being described which is further from a user, while the term "proximal" refers to the portion that is being described which is closer to a user. Further, to the extent consistent, any of the aspects and features detailed herein may be used in conjunction with any or all of the other aspects and features detailed herein.

As used herein, the terms parallel and perpendicular are understood to include relative configurations that are substantially parallel and substantially perpendicular up to about + or - 10 degrees from true parallel and true perpendicular.

"About" or "approximately" or "substantially" as used herein may be inclusive of the stated value and means within an acceptable range of variation for the particular value as determined by one of ordinary skill in the art, considering the measurement in question and the error associated with measurement of the particular quantity (e.g., the limitations of the measurement system).

Descriptions of technical features or aspects of the disclosure should typically be considered as available and applicable to other similar features or aspects of the disclosure. Accordingly, technical features described herein according to one exemplary aspects of the disclosure may be applicable to other exemplary aspects of the disclosure, and thus duplicative descriptions may be omitted herein.

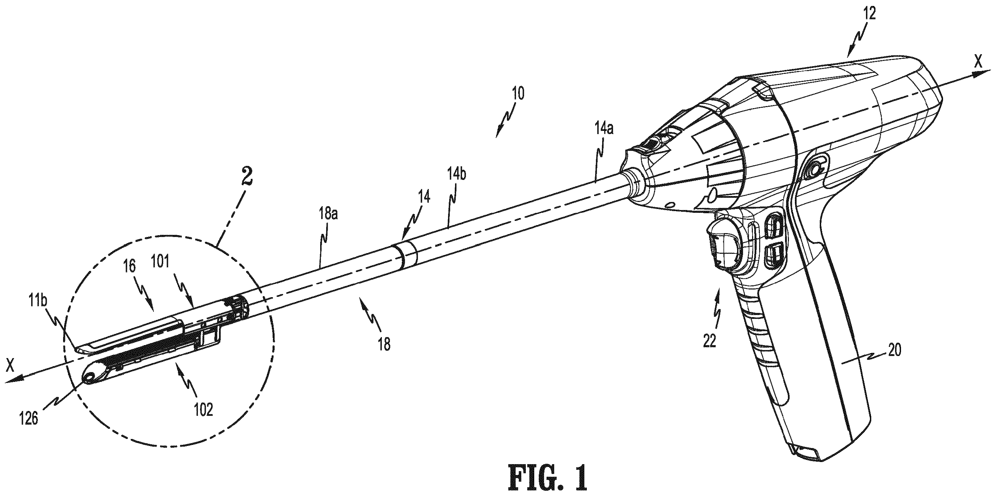

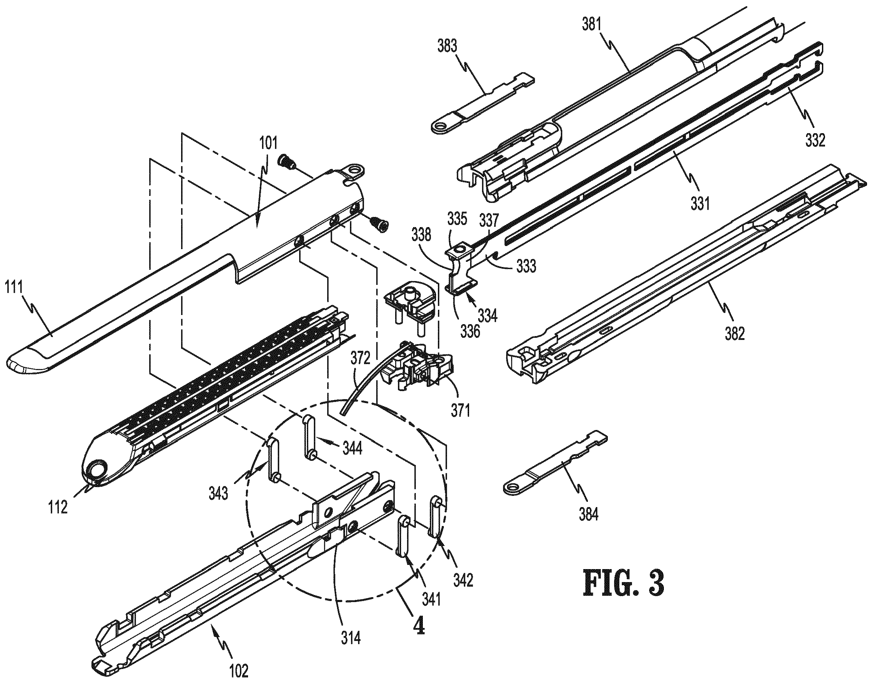

FIGS. 1 and 3 illustrate exemplary aspects of the disclosed surgical stapling device shown as generally as stapling device 10. Stapling device 10 includes a powered handle assembly 12, an elongate body 14, and a tool assembly 16. The elongate body 14 defines a longitudinal axis "X-X" and includes a proximal portion 14a supported on the handle assembly 12 and a distal portion 14b that supports the tool assembly 16. In some aspects of the disclosure, the tool assembly 16 forms part of a reload assembly 18 that includes a proximal body portion 18a that is adapted to be releasably coupled to the distal portion 14b of the elongate body 14 of the stapling device 10. In other aspects of the disclosure, the proximal body portion 18a includes a distal portion that supports the tool assembly 16 for articulation about an axis transverse to the longitudinal axis "X" of the elongate body 14. In alternate aspects of the disclosure, the tool assembly 16 is fixedly secured to the distal portion 14b of the elongate body 14. The proximal body portion 18a may include a first housing member 381, a second housing member 382, a first link 383, and a second link 384 (see FIG. 3). The first and second housing members 381 and 382 are coupled together to form the housing of the proximal body portion 18a. The first and second links 383 and 384 connect the proximal body portion 18a to the tool assembly 16. For a description of exemplary aspects of the proximal body portion and the first and second links, see, e.g., U.S. Patent No. 5,865,361 ("the '361 patent").

The handle assembly 12 of the stapling device 10 includes a stationary handle 20 and actuation buttons 22 that can be depressed to actuate the tool assembly 16, e.g., approximate the tool assembly 16, articulate the tool assembly 16, fire staples, etc. In aspects of the disclosure, batteries (not shown) are supported in the stationary handle 20 to power the handle assembly 12. It is envisioned that the stapling device 10 need not be powered but can also include a manually powered handle assembly such as described in the '361 patent.

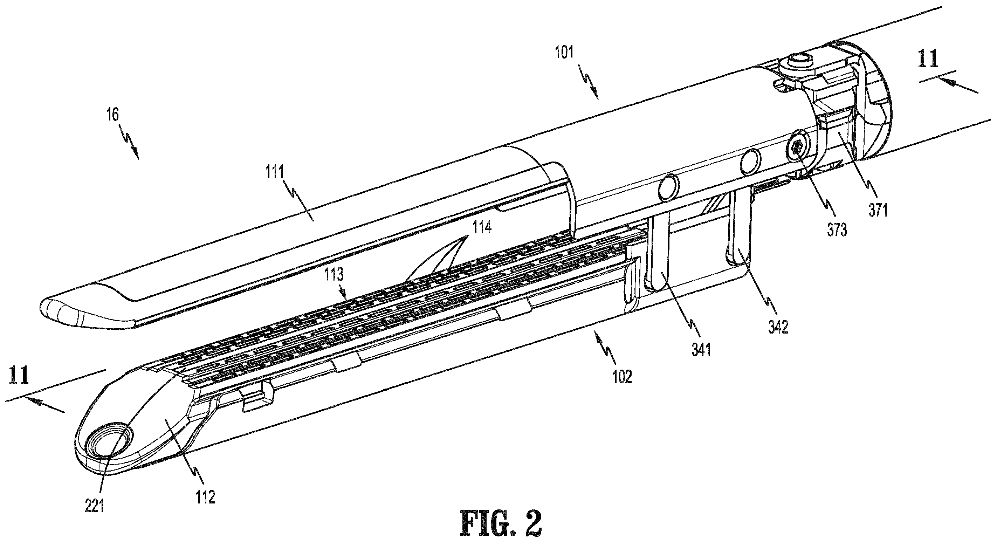

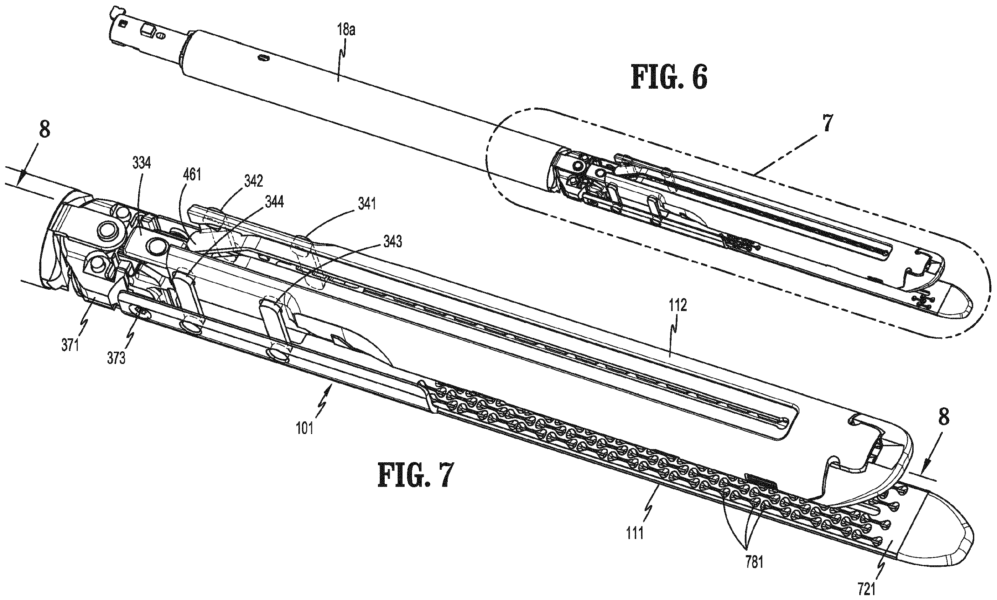

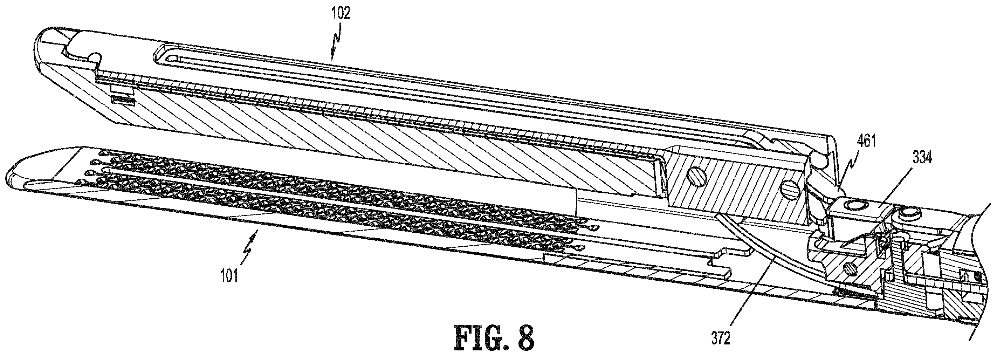

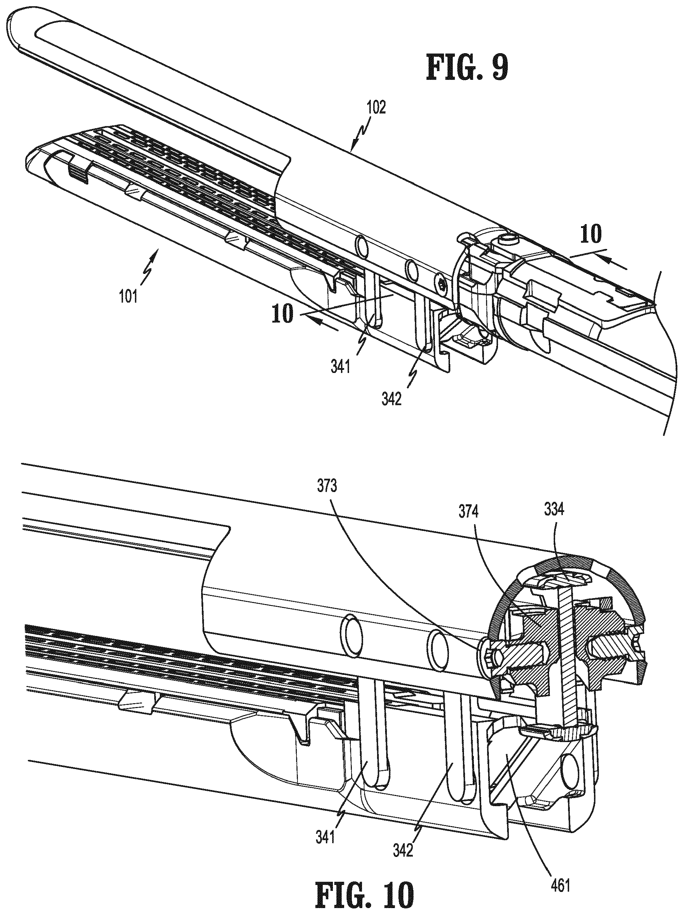

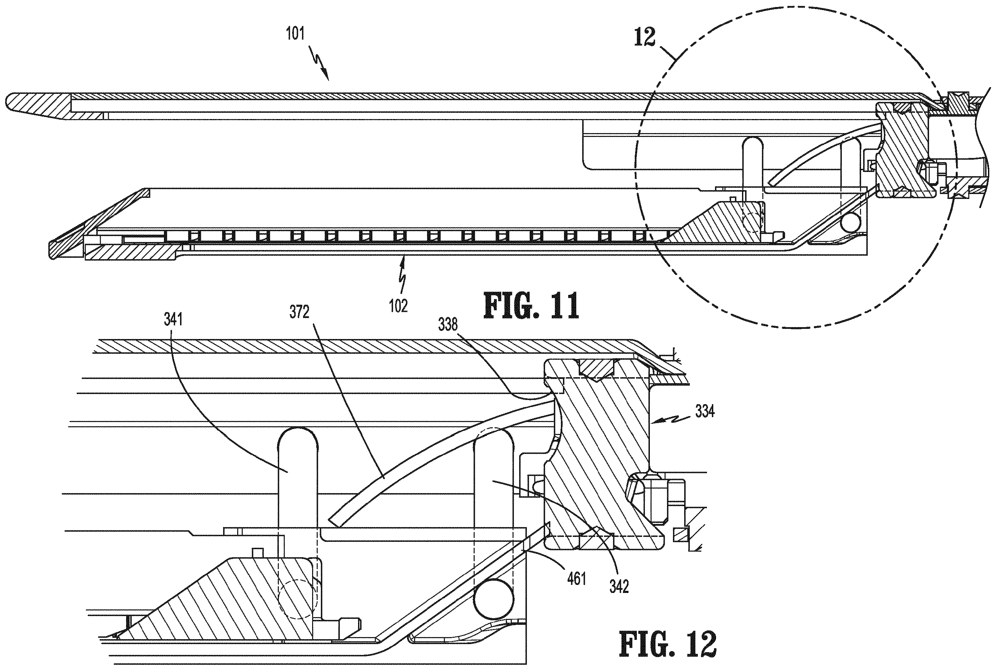

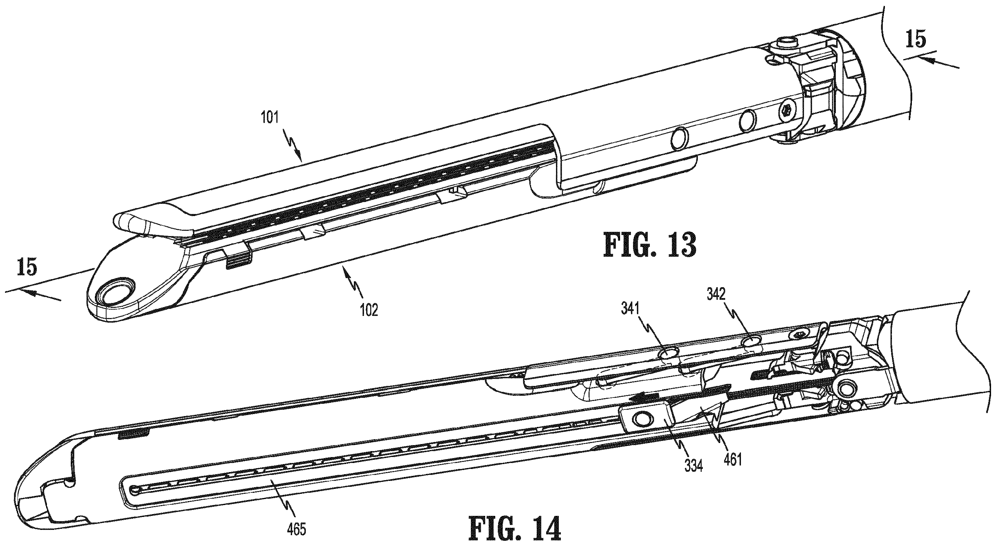

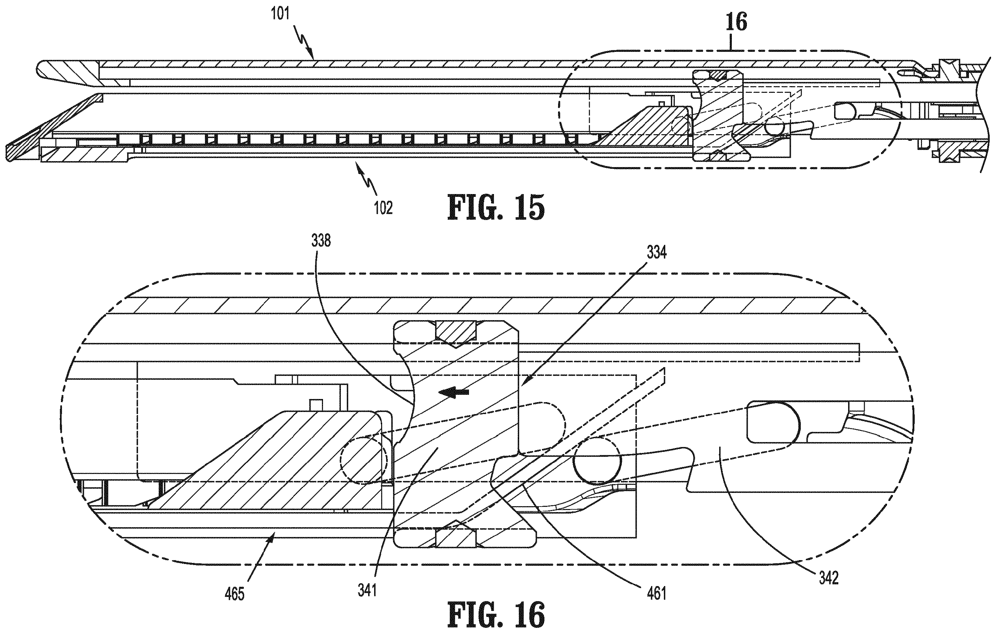

FIGS. 2-16 illustrate the tool assembly 16 for the surgical stapling device 10 of FIG. 1. The tool assembly 16 for the surgical stapling device 10 includes a first jaw 101, a second jaw 102. The first jaw 101 supports an anvil 111 that includes a first surface 721 (see, e.g., FIG. 7). The second jaw 102 supports a cartridge assembly 112 that includes a staple cartridge 113 defining a second surface 221 and a plurality of staple pockets 114. The second jaw 102 is movable in relation to the first jaw 101 between an open position (see, e.g., FIG. 2) and a clamped position (see, e.g., FIG. 13). In the clamped position, the second surface 221 of the staple cartridge 113 is in juxtaposed alignment with the first surface 721 of the anvil 111. In use, the second jaw 102 is moved from the open position to the clamped position by pivoting the second jaw 102 in relation to the first jaw 101 about spaced pivot axes X1 and X2 (FIG. 7) that extend in a direction transverse to the longitudinal axis "X" to move the second jaw 102 distally and toward the first jaw 101, while continuously maintaining parallel alignment between the first surface 721 of the anvil 111 and the second surface 221 of the staple cartridge 113. Thus, a distal-most end 116 of the first jaw 101 is positioned proximal of a distal-most end 126 of the second jaw 102 when the second jaw 102 is in the open position, and the distal-most ends 116 and 126 are gradually brought substantially into vertical alignment with each other as the second jaw 102 moves to the clamped position. When the second jaw 102 is in the clamped position, the first surface 721 of the anvil 111 and the second surface 721 of the staple cartridge 113 define a predetermined minimum tissue gap "G" (see FIG. 15).

A drive member 331 (FIG. 3) has a proximal portion 332 and a distal portion 333. The distal portion 333 of the drive member 331 supports an I-beam 334. The I-beam 334 has a first beam 335, a second beam 336, and a vertical strut 337 connecting the first beam 335 with the second beam 336. The drive member 331 is movable from a retracted position (see, e.g., FIG. 7) towards an advanced position (see, e.g., FIG. 14) to move the second jaw 102 in relation to the first jaw 101 from the open position to the clamped position.

The tool assembly 16 includes two links 341, 342, coupled to a first side of the first and second jaws 101, 102 at longitudinally spaced locations and two links 343, 344, coupled to a second side of the first and second jaws 101, 102 at longitudinally spaced locations. The links 341-344 pivotally connect the second jaw 102 with the first jaw 101. The first pivoting links 341, 342, and the second pivoting links 343, 344, are each configured to pivot in response to longitudinal advancement of the I-beam 334 into engagement with the second jaw 102 to move the second jaw 102 in relation to the first jaw 101 between the open position and the clamped position. Because the first and second jaws 101, 102 are interconnected by two links on each side of the jaws 101, 102, the second surface 221 of the cartridge assembly 112 is maintained in substantially parallel alignment with the first surface 721 of the anvil 111 as the second jaw 102 is moved between the open position and the clamped position. The distance between the first jaw 101 and the second jaw 102 in the open position is determined by the length of the links 341-344 which can be selected to provide a desired gap between the first surface 721 of the anvil 111 and the second surface 221 of the staple cartridge 112.

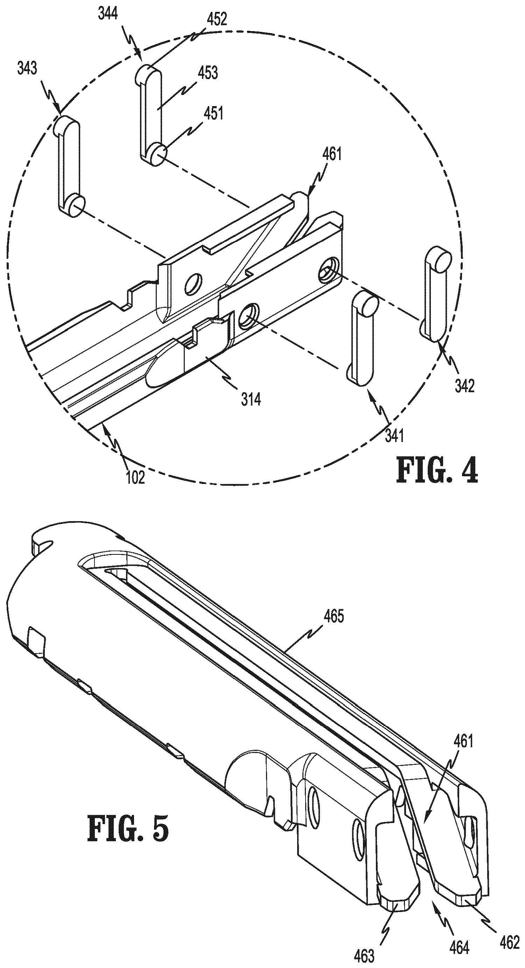

FIG. 4 illustrates the links 341, 342, 343 and 344 that include a first projection 451 pivotably coupled with the second jaw 102 and a second projection 452 pivotably coupled with the first jaw 101. A connecting strut 453 connects the first pivoting projections 451 with the second pivoting projections 452. In aspects of the disclosure, the first pivoting projections 451 may project from the connecting strut 453 in an opposite direction from the second pivoting projections 452. While four links are illustrated (e.g., in FIG. 4) and described herein, additional links having substantially the same structure as described herein may be employed.

Moving the second jaw 102 with respect to the first jaw 101 from the open position to the clamped position while maintaining the continuously parallel arrangement between the first surface 721 of the anvil 111 and the second surface 221 of the cartridge assembly 112 increases the ability of the operator to achieve a desired tissue compression force in the tissue gap "G." Additionally, a pinching of tissue which may occur around the site of a pivot point between an anvil and a cartridge in a conventional tool assembly can be minimized. Maintaining the first surface 721 of the anvil 111 and the second surface 221 of the cartridge assembly 112 in a continuously parallel arrangement applies a more evenly distributed compression force to the tissue during clamping, as compared with a conventional tool assembly employing a pivot point between the anvil 111 and the cartridge assembly 112.

The second jaw 102 of the tool assembly 16 defines a channel 465 including a proximal end portion 314 having a ramp surface 461. A slot 464 extends through the channel 465 and the ramp surface 461. The slot 464 receives the vertical strut 337 of the I-beam 334 as the I-beam 334 translates through the tool assembly 16 when the stapling device 10 is clamped and fired. The channel 465 communicates with the ramped surface 461 and receives and guides the second beam 336 of the I-beam 334 as the I-beam 334 moves along the second jaw 102 during clamping and firing of the stapling device 10. When the second jaw 102 is moved from the open position to the clamped position, the second beam 336 of the I-beam 334 engages the ramped surface 461 to cam the second jaw 102 towards the first jaw 101.

A mounting assembly 371 is secured to the tool assembly 16 and is pivotably coupled to the proximal body portion 18a of the reload assembly 18 to pivotably couple the tool assembly 16 to the proximal body portion 18a of the reload assembly 18. The mounting assembly 371 supports a biasing member, e.g., leaf spring 372, which extends into the tool assembly 16 and urges the second jaw 102 towards the open position. When the second jaw member 102 is moved from the open position to the clamped position as the second beam 336 engages the ramped surface 461 of the second jaw 102, the leaf spring 372 is compressed and deformed. In aspects of the disclosure, the leaf spring 372 is laterally offset from the longitudinal axis "X" so as not to interfere with the movement of the I-Beam 334.

The I-beam 334 may have a distal-facing surface that defines a knife 338 having a sharpened edge configured to cut tissue.

The tool assembly 16 further includes a plurality of staple forming pockets 781 formed in the anvil 111. Each of the staple forming pockets 781 is configured to be in juxtaposed alignment with a corresponding staple pocket 114 of the cartridge assembly 112 when the second jaw 102 is in the clamped position.

The various aspects of the stapling device disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely controls the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the aspects of the disclosure described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A tool assembly (16) for a surgical stapling device, comprising:
a first jaw (101) defining a first surface (721);
a second jaw (102) defining a second surface (221), the second jaw movable in relation to the first jaw between an open position and a clamped position in which the second surface is in juxtaposed alignment with the first surface;
an I-beam (334) movable from a retracted position towards an advanced position to move the second jaw in relation to the first jaw from the open position to the clamped position;
at least two first pivoting links (341, 342) operably coupled to a first side of the first and second jaws; **characterized in** including,
at least two second pivoting links (343, 344) operably coupled to a second side of the first and second jaws, the first pivoting links and the second pivoting links each configured to pivot in response to longitudinal advancement of the I-beam to move the second jaw between the open position and the clamped position,
wherein the second surface of the second jaw is maintained in substantially parallel alignment with the first surface of the first jaw as the second jaw is moved between the open position and the clamped position.

2. The tool assembly of claim 1, wherein each of the first and second pivoting links defines a first pivoting projection (451) engaged with the second jaw, a second pivoting projection (452) engaged with the first jaw, and a connecting strut (453) connecting the first pivoting projection with the second pivoting projection; preferably wherein the first pivoting projection projects in an opposite direction from the second pivoting projection.

3. The tool assembly of any preceding claim, further including a proximally facing ramp surface (461) formed at a proximal end portion of the second jaw, the I-beam engaging the proximal-facing surface of the ramp surface when the I-beam moves from its retracted position towards its advanced position to move the second jaw between the open position and the clamped position,
wherein the ramp surface defines a slot (464) and the I-beam includes a vertical strut, the slot configured to receive the vertical strut of the I-beam.

4. The tool assembly of claim 3, wherein the second jaw defines a channel, the channel (465) configured to receive a lower beam of the I-beam as the I-beam moves the second jaw between the open position and the clamped position.

5. The tool assembly of any preceding claim, further including a mounting assembly secured to the first jaw, the mounting assembly including a leaf spring (372), the leaf spring configured to bias the second jaw towards the open position; preferably wherein the leaf spring is laterally offset from the I-beam.

6. The tool assembly of any preceding claim, wherein the I-beam includes a distal-facing surface that defines a knife (338).

7. A surgical stapling device (10), comprising:
an elongate body (14) having a proximal portion (14a) and a distal portion (14b); and
a reload assembly (18) including a proximal body portion (18a) and a tool assembly (16) as claimed in claim 1, the proximal body portion adapted to be releasably coupled to the distal portion of the elongate body, and the tool assembly being supported on the proximal body portion, wherein:
said first jaw supports an anvil, the anvil defining said first surface;
said second jaw supports a cartridge assembly, the cartridge assembly including a staple cartridge defining said second surface and a plurality of staple pockets, the second jaw movable in relation to the first jaw between an open position and a clamped position in which the second surface of the staple cartridge is in juxtaposed alignment with the first surface of the anvil; and including:
a drive member having a proximal portion and a distal portion, the distal portion of the drive member supporting said I-beam, the I-beam having a first beam, a second beam, and a vertical strut connecting the first beam with the second beam, the drive member movable from a retracted position towards an advanced position to move the second jaw in relation to the first jaw from the open position to the clamped position

8. The surgical stapling device of claim 7, wherein each of the first and pivoting links defines a first pivoting projection engaged with the second jaw, a second pivoting projection engaged with the first jaw, and a connecting strut connecting the first pivoting projection with the second pivoting projection; preferably wherein the first pivoting projection projects in an opposite direction from the second pivoting projection.

9. The surgical stapling device of claim 7 or claim 8, further including a proximally facing ramp surface formed at a proximal end portion of the second jaw, the I-beam engaging the proximal-facing surface of the ramp surface when the I-beam moves from its retracted position towards its advanced position to move the second jaw between the open position and the clamped position,
wherein the ramp surface defines a slot and the I-beam includes a vertical strut, the slot configured to receive the vertical strut of the I-beam; preferably wherein the second jaw defines a channel, the channel configured to receive the lower beam of the I-beam as the I-beam moves the second jaw between the open position and the clamped position.

10. The surgical stapling device of any of claims 7 to 9, further including a mounting assembly secured to the first jaw, the mounting assembly including a leaf spring, the leaf spring configured to bias the second jaw towards the open position; preferably wherein the leaf spring is laterally offset from the I-beam.

11. The surgical stapling device of any of claims 7 to 10, wherein the I-beam includes a distal-facing surface that defines a knife.

12. The surgical stapling device of any of claims 7 to 11, further including a plurality of staple forming pockets formed in the anvil, each of the staple forming pockets configured to be in juxtaposed alignment with a corresponding staple pocket of the cartridge assembly when the second jaw is in the clamped position.

## Patentansprüche

1. Werkzeuganordnung (16) für eine chirurgische Klammervorrichtung, umfassend:
eine erste Backe (101), die eine erste Oberfläche (721) definiert;
eine zweite Backe (102), die eine zweite Oberfläche (221) definiert, wobei die zweite Backe in Bezug auf die erste Backe zwischen einer offenen Position und einer geklemmten Position bewegbar ist, in der die zweite Oberfläche in nebeneinander liegender Ausrichtung mit der ersten Oberfläche ist;
einen I-Träger (334), der aus einer zurückgezogenen Position in eine vorgeschobene Position bewegbar ist, um die zweite Backe in Bezug auf die erste Backe aus der offenen Position in die geklemmte Position zu bewegen;
mindestens zwei erste Schwenkverbindungen (341, 342), die mit einer ersten Seite der ersten und der zweiten Klemmbacke wirkgekoppelt sind; **dadurch gekennzeichnet, dass** sie Folgendes einschließen:
mindestens zwei zweite Schwenkverbindungen (343, 344), die mit einer zweiten Seite der ersten und der zweiten Klemmbacke wirkgekoppelt sind, wobei die ersten Schwenkverbindungen und die zweiten Schwenkverbindungen jeweils dazu ausgelegt sind, in Reaktion auf Längsvorschub des I-Trägers zu schwenken, um die zweite Klemmbacke zwischen der offenen Position und der geklemmten Position zu bewegen,
wobei die zweite Oberfläche der zweiten Backe in im Wesentlichen paralleler Ausrichtung mit der ersten Oberfläche der ersten Backe gehalten wird, während die zweite Backe zwischen der offenen Position und der geklemmten Position bewegt wird.

2. Werkzeuganordnung nach Anspruch 1, wobei jede der ersten und zweiten Schwenkverbindungen einen ersten Schwenkvorsprung (451), der mit der zweiten Backe in Eingriff steht, einen zweiten Schwenkvorsprung (452), der mit der ersten Backe in Eingriff steht, und eine Verbindungsstrebe (453) definiert, die den ersten Schwenkvorsprung mit dem zweiten Schwenkvorsprung verbindet; wobei vorzugsweise der erste Schwenkvorsprung in eine entgegengesetzte Richtung zu dem zweiten Schwenkvorsprung vorsteht.

3. Werkzeuganordnung nach einem der vorhergehenden Ansprüche, die ferner eine nach proximal weisende Rampenoberfläche (461) einschließt, die an einem proximalen Endabschnitt der zweiten Backe gebildet ist, wobei der I-Träger mit der nach proximal weisenden Oberfläche der Rampenoberfläche in Eingriff steht, wenn sich der I-Träger aus seiner zurückgezogenen Position in seine vorgeschobene Position bewegt, um die zweite Backe zwischen der offenen Position und der geklemmten Position zu bewegen,
wobei die Rampenoberfläche einen Schlitz (464) definiert und der I-Träger eine vertikale Strebe einschließt, wobei der Schlitz dazu ausgelegt ist, die vertikale Strebe des I-Trägers aufzunehmen.

4. Werkzeuganordnung nach Anspruch 3, wobei die zweite Backe einen Kanal definiert, wobei der Kanal (465) dazu ausgelegt ist, einen unteren Träger des I-Trägers aufzunehmen, wenn der I-Träger die zweite Backe zwischen der offenen Position und der geklemmten Position bewegt.

5. Werkzeuganordnung nach einem der vorhergehenden Ansprüche, die ferner eine Montageanordnung einschließt, die an der ersten Backe befestigt ist, wobei die Montageanordnung eine Blattfeder (372) einschließt, wobei die Blattfeder dazu ausgelegt ist, die zweite Backe in Richtung der offenen Position vorzuspannen; wobei die Blattfeder vorzugsweise lateral zu dem I-Träger versetzt ist.

6. Werkzeuganordnung nach einem der vorhergehenden Ansprüche, wobei der I-Träger eine nach distal weisende Oberfläche einschließt, die eine Klinge (338) definiert.

7. Chirurgische Klammervorrichtung (10), umfassend:
einen länglichen Körper (14) mit einem proximalen Abschnitt (14a) und einem distalen Abschnitt (14b); und
eine Nachladeanordnung (18), die einen proximalen Körperabschnitt (18a) und eine Werkzeuganordnung (16) nach Anspruch 1 einschließt, wobei der proximale Körperabschnitt dazu eingerichtet ist, lösbar mit dem distalen Abschnitt des länglichen Körpers gekoppelt zu werden, und wobei die Werkzeuganordnung auf dem proximalen Körperabschnitt getragen wird, wobei:
die erste Backe einen Amboss trägt, wobei der Amboss die erste Oberfläche definiert;
die zweite Backe eine Magazinanordnung trägt, wobei die Magazinanordnung ein Klammermagazin einschließt, welches die zweite Oberfläche und eine Vielzahl von Klammertaschen definiert, wobei die zweite Backe in Bezug auf die erste Backe zwischen einer offenen Position und einer geklemmten Position, in der die zweite Oberfläche des Klammermagazins in nebeneinander liegender Ausrichtung mit der ersten Oberfläche des Ambosses ist, bewegbar ist; und Folgendes einschließt:
ein Antriebselement mit einem proximalen Abschnitt und einem distalen Abschnitt, wobei der distale Abschnitt des Antriebselements den I-Träger trägt, wobei der I-Träger einen ersten Träger, einen zweiten Träger und eine vertikale Strebe aufweist, die den ersten Träger mit dem zweiten Träger verbindet, wobei das Antriebselement aus einer zurückgezogenen Position in eine vorgeschobene Position bewegbar ist, um die zweite Backe in Bezug auf die erste Backe aus der offenen Position in die geklemmte Position zu bewegen.

8. Chirurgische Klammervorrichtung nach Anspruch 7, wobei jede der ersten und Schwenkverbindungen einen ersten Schwenkvorsprung, der mit der zweiten Backe in Eingriff steht, einen zweiten Schwenkvorsprung, der mit der ersten Backe in Eingriff steht, und eine Verbindungsstrebe definiert, welche den ersten Schwenkvorsprung mit dem zweiten Schwenkvorsprung verbindet; wobei vorzugsweise der erste Schwenkvorsprung in eine entgegengesetzte Richtung zu dem zweiten Schwenkvorsprung vorsteht.

9. Chirurgische Klammervorrichtung nach Anspruch 7 oder Anspruch 8, die ferner eine nach proximal weisende Rampenoberfläche einschließt, die an einem proximalen Endabschnitt der zweiten Backe gebildet ist, wobei der I-Träger mit der nach proximal weisenden Oberfläche der Rampenoberfläche in Eingriff steht, wenn sich der I-Träger aus seiner zurückgezogenen Position in seine vorgeschobene Position bewegt, um die zweite Backe zwischen der offenen Position und der geklemmten Position zu bewegen,
wobei die Rampenoberfläche einen Schlitz definiert und der I-Träger eine vertikale Strebe einschließt, wobei der Schlitz dazu ausgelegt ist, die vertikale Strebe des I-Trägers aufzunehmen; wobei vorzugsweise die zweite Backe einen Kanal definiert, wobei der Kanal dazu ausgelegt ist, den unteren Träger des I-Trägers aufzunehmen, wenn der I-Träger die zweite Backe zwischen der offenen Position und der geklemmten Position bewegt.

10. Chirurgische Klammervorrichtung nach einem der Ansprüche 7 bis 9, die ferner eine Montageanordnung umfasst, die an der ersten Backe befestigt ist, wobei die Montageanordnung eine Blattfeder einschließt, wobei die Blattfeder dazu ausgelegt ist, die zweite Backe in Richtung der offenen Position vorzuspannen; wobei die Blattfeder vorzugsweise lateral zu dem I-Träger versetzt ist.

11. Chirurgische Klammervorrichtung nach einem der Ansprüche 7 bis 10, wobei der I-Träger eine nach distal weisende Oberfläche einschließt, die eine Klinge definiert.

12. Chirurgische Klammervorrichtung nach einem der Ansprüche 7 bis 11, die ferner eine Vielzahl von Klammerformungstaschen einschließt, die in dem Amboss gebildet sind, wobei jede der Klammerformungstaschen dazu ausgelegt ist, sich in nebeneinander liegender Ausrichtung mit einer entsprechenden Klammertasche der Magazinanordnung zu befinden, wenn sich die zweite Backe in der geklemmten Position befindet.

## Revendications

1. Ensemble d'outil (16) pour un dispositif d'agrafage chirurgical, comprenant :
une première mâchoire (101) définissant une première surface (721) ;
une seconde mâchoire (102) définissant une seconde surface (221), la seconde mâchoire étant mobile par rapport à la première mâchoire entre une position ouverte et une position serrée dans laquelle la seconde surface est en alignement juxtaposé avec la première surface ;
une poutre en I (334) mobile d'une position rétractée vers une position avancée pour déplacer la seconde mâchoire par rapport à la première mâchoire de la position ouverte à la position serrée ;
au moins deux premières liaisons pivotantes (341, 342) couplées fonctionnellement à un premier côté des première et seconde mâchoires ; **caractérisé en ce qu'**il comprend, au moins deux secondes liaisons pivotantes (343, 344) couplées fonctionnellement à un second côté des première et seconde mâchoires, les premières liaisons pivotantes et les secondes liaisons pivotantes étant chacune configurées pour pivoter en réponse à l'avancement longitudinal de la poutre en I pour déplacer la seconde mâchoire entre la position ouverte et la position serrée, la seconde surface de la seconde mâchoire étant maintenue en alignement sensiblement parallèle avec la première surface de la première mâchoire lorsque la seconde mâchoire est déplacée entre la position ouverte et la position serrée.

2. Ensemble d'outil selon la revendication 1, chacune des première et seconde liaisons pivotantes définissant une première saillie pivotante (451) en prise avec la seconde mâchoire, une seconde saillie pivotante (452) en prise avec la première mâchoire, et une entretoise de connexion (453) reliant la première saillie pivotante à la seconde saillie pivotante ; de préférence, la première saillie pivotante faisant saillie dans une direction opposée à la seconde saillie pivotante.

3. Ensemble d'outil selon l'une quelconque des revendications précédentes, comprenant en outre une surface de rampe (461) orientée vers le côté proximal formée au niveau d'une partie d'extrémité proximale de la seconde mâchoire, la poutre en I venant en prise avec la surface orientée vers le côté proximal de la surface de rampe lorsque la poutre en I se déplace de sa position rétractée vers sa position avancée pour déplacer la seconde mâchoire entre la position ouverte et la position serrée,
la surface de rampe définissant une fente (464) et la poutre en I comprenant une entretoise verticale, la fente étant configurée pour recevoir l'entretoise verticale de la poutre en I.

4. Ensemble d'outil selon la revendication 3, la seconde mâchoire définissant un canal, le canal (465) étant configuré pour recevoir une poutre inférieure de la poutre en I lorsque la poutre en I déplace la seconde mâchoire entre la position ouverte et la position serrée.

5. Ensemble d'outil selon l'une quelconque des revendications précédentes, comprenant en outre un ensemble de montage fixé à la première mâchoire, l'ensemble de montage comprenant un ressort à lame (372), le ressort à lame étant configuré pour solliciter la seconde mâchoire vers la position ouverte ; de préférence, le ressort à lame étant décalé latéralement par rapport à la poutre en I.

6. Ensemble d'outil selon l'une quelconque des revendications précédentes, la poutre en I comprenant une surface orientée vers le côté distal qui définit un couteau (338).

7. Dispositif d'agrafage chirurgical (10), comprenant :
un corps allongé (14) ayant une partie proximale (14a) et une partie distale (14b) ; et
un ensemble de rechargement (18) comprenant une partie de corps proximale (18a) et un ensemble d'outil (16) selon la revendication 1, la partie de corps proximale étant adaptée pour être couplée de manière libérable à la partie distale du corps allongé, et l'ensemble d'outil étant supporté sur la partie de corps proximale,
ladite première mâchoire supportant une enclume, l'enclume définissant ladite première surface ;
ladite seconde mâchoire supportant un ensemble cartouche, l'ensemble cartouche comprenant une cartouche d'agrafes définissant ladite seconde surface et une pluralité de poches d'agrafes, la seconde mâchoire étant mobile par rapport à la première mâchoire entre une position ouverte et une position serrée dans laquelle la seconde surface de la cartouche d'agrafes est en alignement juxtaposé avec la première surface de l'enclume ; et comprenant :
un élément d'entraînement ayant une partie proximale et une partie distale, la partie distale de l'élément d'entraînement supportant ladite poutre en I, la poutre en I ayant une première poutre, une seconde poutre, et une entretoise verticale reliant la première poutre à la seconde poutre, l'élément d'entraînement étant mobile d'une position rétractée vers une position avancée pour déplacer la seconde mâchoire par rapport à la première mâchoire de la position ouverte à la position serrée.

8. Dispositif d'agrafage chirurgical selon la revendication 7, chacune des première et liaisons pivotantes définissant une première saillie pivotante en prise avec la seconde mâchoire, une seconde saillie pivotante en prise avec la première mâchoire, et une entretoise de connexion reliant la première saillie pivotante à la seconde saillie pivotante ; de préférence, la première saillie pivotante faisant saillie dans une direction opposée à la seconde saillie pivotante.

9. Dispositif d'agrafage chirurgical selon la revendication 7 ou la revendication 8, comprenant en outre une surface de rampe orientée vers le côté proximal formée au niveau d'une partie d'extrémité proximale de la seconde mâchoire, la poutre en I venant en prise avec la surface orientée vers le côté proximal de la surface de rampe lorsque la poutre en I se déplace de sa position rétractée vers sa position avancée pour déplacer la seconde mâchoire entre la position ouverte et la position serrée,
la surface de rampe définissant une fente et la poutre en I comprenant une entretoise verticale, la fente étant configurée pour recevoir l'entretoise verticale de la poutre en I ; de préférence, la seconde mâchoire définissant un canal, le canal étant configuré pour recevoir la poutre inférieure de la poutre en I lorsque la poutre en I déplace la seconde mâchoire entre la position ouverte et la position serrée.

10. Dispositif d'agrafage chirurgical selon l'une quelconque des revendications 7 à 9, comprenant en outre un ensemble de montage fixé à la première mâchoire, l'ensemble de montage comprenant un ressort à lame, le ressort à lame étant configuré pour solliciter la seconde mâchoire vers la position ouverte ; de préférence, le ressort à lame étant décalé latéralement par rapport à la poutre en I.

11. Dispositif d'agrafage chirurgical selon l'une quelconque des revendications 7 à 10, la poutre en I comprenant une surface orientée vers le côté distal qui définit un couteau.

12. Dispositif d'agrafage chirurgical selon l'une quelconque des revendications 7 à 11, comprenant en outre une pluralité de poches de formation d'agrafes formées dans l'enclume, chacune des poches de formation d'agrafes étant configurée pour être en alignement juxtaposé avec une poche d'agrafes correspondante de l'ensemble cartouche lorsque la seconde mâchoire est dans la position serrée.
